# FASCICULE DE BREVET EUROPEEN

(11) **EP 3 645 312 B1**
(45) Date de publication et mention de la délivrance du brevet: **19.05.2021**
(21) Numéro de dépôt: 18758912.2
(22) Date de dépôt: 28.06.2018
(51) Int. Cl.: B60C 1/00, C08F 236/06, C08F 236/08

(54) **PROCEDE DE PREPARATION D'UN COPOLYMERE STATISTIQUE A BASE DE MONOMERE DIENIQUE ACYCLIQUE ET DE MONOMERE DIENIQUE CYCLIQUE, COPOLYMERES ET COMPOSITIONS LES COMPRENANT**
VERFAHREN ZUR HERSTELLUNG EINES STATISTISCHEN COPOLYMERS AUF BASIS VON ACYCLISCHEN DIENMONOMEREN UND CYCLISCHEN DIENMONOMEREN, COPOLYMERE UND ZUSAMMENSETZUNGEN DAMIT
PROCESS FOR PREPARING A STATISTICAL COPOLYMER BASED ON ACYCLIC DIENE MONOMERS AND CYCLIC DIENE MONOMERS, COPOLYMERS AND COMPOSITIONS CONTAINING SAME

(30) Priorité: 30.06.2017 FR 1756120
(43) Date de publication de la demande: 06.05.2020
(73) Titulaire: Compagnie Générale des Etablissements Michelin, 63000 Clermont-Ferrand (FR)
(72) Inventeur: MATMOUR, Rachid, 63040 Clermont-Ferrand Cedex 9 (FR); NGO, Robert, 63040 Clermont-Ferrand Cedex 9 (FR); SCHNELL, Benoît, 63040 Clermont-Ferrand Cedex 9 (FR)
(74) Mandataire: Le Cam, Véronique Marie Christine
(86) Numéro de dépôt international: PCT/FR2018/051584
(87) Numéro de publication internationale: WO 2019/002770

(56) Documents cités:
- EP-A1- 1 829 906
- WO-A1-2014/053509
- US-A- 3 935 177
- ITARU NATORI AND SHOHEI INOUE: "Living Anionic Polymerization of 1,3-Cyclohexadiene with the n-Butyllithium/N,N,N,N-Tetramethylethylene diamine System. Copolymerization and Block Copolymerization with Styrene, Butadiene, and Isoprene", MACROMOLECULES, vol. 4, no. 31, 31 janvier 1998 (1998-01-31), pages 982-987, XP002779116, DOI: 10.1021/ma9712110

## Description

La présente invention concerne un procédé de synthèse de copolymères statistiques comprenant au sein de la chaine polymérique principale des cycles comprenant au moins une double liaison, de tels copolymères et les compositions les comprenant.

La structure chimique d'un polymère impacte généralement les propriétés chimiques et physiques du polymère, ainsi que les propriétés des compositions le contenant.

La Demanderesse a ainsi cherché à copolymériser des monomères diéniques acycliques, tels que le 1,3-butadiène, avec des monomères cycliques. On recherche particulièrement des copolymères statistiques. Lors de ses recherches, la Demanderesse a constaté qu'avec un monomère tel que le cyclohexadiène, les essais de copolymérisation avec le 1,3-butadiène ou l'isoprène n'ont pas conduit à des copolymères statistiques, la formation de blocs étant favorisée, ou les réactions secondaires étant trop importantes. Ces observations sont venues confirmer les résultats reportés par Natori (Macromolecules 1998, 31, 982-987) démontrant que lors de la copolymérisation de cyclohexadiène et d'isoprène ou de cyclohexadiène et de styrène, l'incorporation de l'isoprène ou du styrène est beaucoup plus rapide que celle du cyclohexadiène et aboutit à l'obtention de copolymères diblocs.

Poursuivant ses recherches, la Demanderesse a découvert qu'il était possible d'insérer statistiquement des unités cycliques comprenant au moins une double liaison à partir de monomères particuliers et dans des conditions opératoires particulières.

Ainsi, un premier objet de l'invention est un procédé de préparation d'un copolymère statistique à base de monomères diéniques acycliques et de monomères diéniques cycliques, caractérisé en ce qu'il comprend une étape de copolymérisation, en présence d'un agent polaire et d'un amorceur anionique dans un solvant de polymérisation, d'au moins un monomère diénique acyclique et d'au moins un monomère diénique cyclique dont une double liaison C=C est endocyclique et conjuguée à une double liaison C=C exocyclique, à une température de polymérisation inférieure à 80°C, le rapport molaire [agent polaire/fonction de l'amorceur anionique] étant supérieur à 0,1.

En particulier, le rapport molaire [agent polaire/ fonction de l'amorceur anionique] est supérieur ou égal à 0,3.

Avantageusement, le monomère diénique acyclique est un monomère diénique conjugué, de préférence le butadiène-1,3 ou l'isoprène.

Avantageusement, le monomère diénique cyclique est un monomère répondant à la formule (I) suivante : 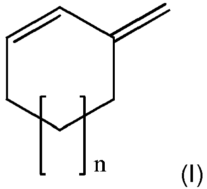 Où n=0 ou 1,
de préférence le 3-méthylènecyclopentène.

En particulier, dans le procédé selon la présente invention, on copolymérise également un composé vinylaromatique ayant de 8 à 20 atomes de carbone, avantageusement le styrène.

Avantageusement, l'agent polaire est une tétraalkyldiamine, notamment, la N,N,N',N'-tétraméthyléthylènediamine.

Avantageusement, l'amorceur anionique contient un métal alcalin, plus avantageusement l'amorceur anionique est un organolithien.

En particulier, la température de polymérisation varie de 45°C à 70°C, de préférence de 50°C à 60°C.

L'invention a également pour objet un copolymère statistique comprenant, réparties de manière statistique au sein de la chaine linéaire principale du copolymère, des unités insaturées issues d'au moins un monomère diénique acyclique et des unités cycliques issues d'au moins un monomère diénique cyclique dont une double liaison C=C est endocyclique et conjuguée à une double liaison C=C exocyclique.

En particulier, le monomère diénique acyclique est un monomère diénique conjugué, de préférence le butadiène-1,3 ou l'isoprène.

En particulier, le monomère diénique cyclique est un monomère répondant à la formule (I) suivante : 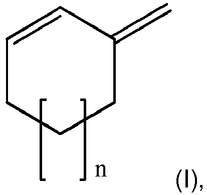 de préférence le 3-méthylènecyclopentène.

Avantageusement, le copolymère selon l'invention comprend également des unités issues d'un composé vinylaromatique ayant de 8 à 20 atomes de carbone, avantageusement du styrène.

Avantageusement, le pourcentage molaire d'unités issues du monomère diénique cyclique, par rapport au nombre total d'unités, est d'au moins 5%, avantageusement d'au moins 10%.

En particulier, le pourcentage molaire d'unités issues du monomère diénique cyclique, par rapport au nombre total d'unités, est inférieur à 50%, avantageusement inférieur à 30%.

L'invention a également pour objet un copolymère susceptible d'être obtenu par le procédé selon l'invention.

En particulier, le copolymère selon l'invention est un élastomère.

L'invention a également pour objet une composition comprenant le copolymère selon l'invention.

L'invention a également pour objet un pneumatique dont un de ses éléments constitutifs comprend une composition selon l'invention.

Dans la présente description, tout intervalle de valeurs désigné par l'expression « de a à b » représente le domaine de valeurs allant de a jusqu'à b (c'est-à-dire bornes a et b inclues). Tout intervalle « entre a et b » représente le domaine de valeurs allant de plus de a à moins de b (c'est-à-dire bornes a et b exclues).

Par « unité insaturée », on entend une unité issue d'un monomère diène et comportant une double liaison.

Par « distribution statistique », on entend une distribution des unités constitutives du copolymère obéissant à une loi statistique.

Par « agent polaire », on entend une molécule dans laquelle la répartition des charges partielles n'est pas symétrique, induisant un moment dipolaire dans la molécule.

Par « fonction de l'amorceur anionique », on entend la ou les fonctions apte(s) à amorcer la polymérisation anionique. Lorsque l'amorceur anionique est un composé contenant un métal alcalin, chaque métal alcalin est une fonction de l'amorceur anionique.

Par « cycle comprenant au moins une double liaison », on entend un cycle comprenant au moins une double liaison C=C endocyclique ou exocyclique.

Le monomère diénique cyclique dont une double liaison C=C est endocyclique et conjuguée à une double liaison C=C exocyclique pourra également être désigné, dans le cadre de la présente description, par les termes « monomère diénique cyclique ».

Les composés mentionnés dans la description et entrant dans la préparation de polymères ou de compositions de caoutchouc peuvent être d'origine fossile ou biosourcés. Dans ce dernier cas, ils peuvent être, partiellement ou totalement, issus de la biomasse ou obtenus à partir de matières premières renouvelables issues de la biomasse. Sont concernés notamment les monomères.

### I- Description détaillée de l'invention

### I.1 Procédé selon l'invention

### 1.1.1 Monomères

Dans le procédé selon l'invention, on met en œuvre au moins un monomère diénique acyclique et au moins un monomère diénique cyclique dont une double liaison C=C est endocyclique et conjuguée à une double liaison C=C exocyclique.

Selon l'invention, ces monomères s'insèrent de manière statistique.

Selon l'invention, le monomère diène s'insère selon un enchainement de type 1,4 ou de type 1,2, ou sous la forme d'un cycle, avantageusement de manière aléatoire et indépendante.

A titre de monomère diénique acyclique, on peut utiliser selon le procédé conforme à l'invention tout monomère diène conjugué ayant 4 à 12 atomes de carbone. Le 1,3-butadiène, le 2-méthyl-1,3-butadiène (encore dénommé isoprène), les 2,3-di(alkyle en C1 à C5)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, le phényl-1,3-butadiène, le 1,3-pentadiène, le 2,4-hexadiène, etc. ainsi que leurs mélanges conviennent. Le monomère diénique acyclique est de préférence le 1,3-butadiène ou l'isoprène.

A titre de monomère diénique cyclique, on peut utiliser selon le procédé conforme à l'invention tout monomère diène conjugué cyclique dont une double liaison C=C est endocyclique et conjuguée à une double liaison C=C exocyclique.

En particulier, le monomère répond à la formule (I) suivante :

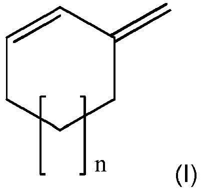

Où n = 0 ou 1, avantageusement n=0.

Le monomère diénique cyclique est de préférence le 3-méthylènecyclopentène.

Le diène conjugué cyclique s'insère sous la forme d'un cycle comprenant une double liaison endocyclique ou sous la forme d'un cycle comprenant une double liaison exocyclique.

Outre ces monomères, on peut également copolymériser au moins un autre monomère. Cet autre monomère peut être un composé vinylaromatique ayant de 8 à 20 atomes de carbone. A titre de composé vinylaromatique convient le styrène, l'ortho-, méta-, para-méthylstyrène, le mélange commercial « vinyltoluène », le para-tertiobutylstyrène, les méthoxystryrènes, le vinylmésitylène, le divinybenzène, le vinylnaphtalène. Cet autre monomère est de préférence le styrène.

Les monomères copolymérisent par polymérisation anionique.

Le mélange réactionnel comprend avantageusement, par rapport au poids total des monomères, au moins 20% en masse de monomère diénique cyclique. Le mélange réactionnel pourra avantageusement comprendre jusqu'à 55% en masse de monomère diénique cyclique, par rapport au poids total des monomères.

Selon une variante de l'invention, le mélange réactionnel comprend, par rapport au poids total des monomères, de 25% à 45% en masse de monomère diénique cyclique.

Selon une autre variante de l'invention, le mélange réactionnel comprend, par rapport au poids total des monomères, de 45% à 55% en masse de monomère diénique cyclique.

Le mélange réactionnel comprend avantageusement, par rapport au poids total des monomères, au moins 45% en masse de monomère diénique acyclique. Le mélange réactionnel pourra avantageusement comprendre jusqu'à 80% en masse de monomère diénique acyclique, par rapport au poids total des monomères.

Selon une variante de l'invention, le mélange réactionnel comprend, par rapport au poids total des monomères, de 55% à 75% en masse de monomère diénique acyclique.

Selon une autre variante de l'invention, le mélange réactionnel comprend, par rapport au poids total des monomères, de 45% à 55% en masse de monomère diénique acyclique.

Outre les monomères mis en jeu, le procédé selon l'invention se caractérise également par la présence d'un agent polaire.

### 1.1.2 Agent polaire et amorceur anionique

La présence d'un agent polaire est obligatoire pour obtenir le copolymère recherché. Ainsi, dans le procédé selon l'invention, le rapport molaire [agent polaire/fonction de l'amorceur anionique] est supérieur à 0,1, avantageusement supérieur à 0,3, plus avantageusement supérieur à 0,4. Le rapport molaire [agent polaire/fonction de l'amorceur anionique] peut aller jusqu'à 3, avantageusement jusqu'à 2.

Tout type d'agent polaire peut être utilisé.

En particulier, l'agent polaire peut être un agent polaire non chélatant ou un agent polaire chélatant. L'agent polaire chélatant est un composé organique qui possède sur au moins deux atomes au moins un doublet non liant. Au contraire, un agent polaire non chélatant est un composé organique qui possède au plus un doublet non liant.

Notamment, à titre d'agents polaires non chélatants, on peut citer des bases de Lewis de type éther, amines tertiaires ou les disulfures. En particulier, on peut utiliser des éthers tels que le diéthyl éther, le di-n-propyl éther, le diisopropyl éther, le diphényl éther, l'anisole ou encore des éthers cycliques tels que le tétrahydrofurane ou le dioxane. On peut également utiliser des amines tertiaires en tant qu'agent polaire non chélatant, telles que des trialkylamines, notamment la triméthylamine ou la triéthylamine, des amines cycliques, notamment la N-méthyl morpholine ou la N-éthyl morpholine.

Notamment, les agents polaires chélatants peuvent être utilisés. En particulier, on peut utiliser les polyéthers tels que l'éthylène glycol diméthyl éther, l'éthylène glycol diéthyl éther, le diéthylène glycol diméthyl éther ou diglyme, le diéthylène glycol diéthyl éther ou le triéthylène glycol diméthyl éther ou triglyme. On peut également utiliser des tétraalkyldiamines, notamment, la N,N,N',N'-tétraméthyléthylènediamine ou TMEDA, le 1,4-Diazabicyclo[2.2.2]octane ou DABCO, ou utiliser des dérivés phosphorés tels que l'hexaméthylphosphoramide ou HMPA. De préférence, l'agent polaire est une tétraalkyldiamine.

En tant qu'amorceur de polymérisation, on peut utiliser tout amorceur anionique monofonctionnel ou polyfonctionnel connu. Toutefois un amorceur contenant un métal alcalin tel que le lithium, le sodium ou le potassium est utilisé à titre préférentiel, et tout particulièrement un amorceur contenant du lithium.

Comme amorceurs organolithiens conviennent notamment ceux comportant au moins une liaison carbone-lithium ou au moins une liaison azote-lithium. Le nombre de liaisons carbone-lithium ou azote-lithium peut varier jusqu'à 12 liaisons, plus particulièrement jusqu'à 4 liaisons.

Comme amorceurs organolithiens comportant une ou plusieurs liaisons carbone-lithium conviennent par exemple les organolithiens aliphatiques tels que l'éthyllithium, le n-butyllithium (n-BuLi), le s-butyllithium (s-BuLi), l'isobutyllithium, les polyméthylènes dilithium tels que le 1,4-dilithiobutane, le 1,4-dilithiotétraphénylbutane, les amorceurs dilithiés issus de la réaction d'un organolitien sur un composé divinyl aliphatique ou un composé divinyl aromatique, notamment la réaction de s-butyllithium sur les dialcénylbenzenes, tels que le 1,3-diisopropylbenzène ou le divinylbenzène tels que décrits dans le brevet EP1237941 B1. Parmi les organolithiens comportant plusieurs liaisons carbone-lithium, l'adduit de réaction entre le 1,3-diisopropénylbenzene et le s-butyl lithium est préféré.

Comme amorceurs organolithiens comportant une ou plusieurs liaisons azote-lithium conviennent par exemple les amidures de lithium issus de la réaction d'un organolithien sur une amine secondaire acyclique ou cyclique, de préférence cyclique, des amidures de dilithium issus de la réaction d'un organolithien sur un composé comprenant deux groupements azotés par exemple pyridinyle, tels que le 1,8-bis(6-ethyl-2-pyridyl)-octane dilithium issu de la réaction du 1,8-bis(6-ethyl-2-pyridyl)-octane avec le tert-butyllithium.

A titre d'amine secondaire utilisable pour préparer les amorceurs, on peut citer la diméthylamine, diéthylamine, dipropylamine, di-n-butylamine, di-sec-butylamine, dipentylamine, dihexylamine, di-n-octylamine, di-(2-ethylhexyl)amine, di-cyclohexylamine, N-méthylbenzylamine, diallylamine, morpholine, piperazine, 2,6-diméthylmorpholine, 2,6-diméthylpiperazine, 1-éthylpiperazine, 2-méthylpiperazine, 1-benzylpiperazine, piperidine, 3,3-diméthylpiperidine, 2,6-dimethylpiperidine, 1-méthyl-4-(méthylamino)piperidine, 2,2,6,6-tetraméthylpiperidine, pyrrolidine, 2,5-diméthylpyrrolidine, azetidine, hexaméthylèneimine, heptaméthylèneimine, 5-benzyloxyindole, 3-azaspiro[5,5]undecane, 3-aza-bicycle[3.2.2]nonane, carbazole, bistriméthylsilylamine, la pyrrolidine et l'hexaméthylèneamine. L'amine secondaire, lorsqu'elle est cyclique, est de préférence choisie parmi la pyrrolidine et l'hexaméthylèneamine.

Le composé organolitien est de préférence l'éthyllithium, le n-butyllithium (n-BuLi), le s-butyllithium (s-BuLi), l'isobutyllithium, etc.

Le rapport molaire [agent polaire/métal alcalin de l'amorceur anionique] est supérieur à 0,1, avantageusement supérieur à 0,3, plus avantageusement supérieur à 0,4. Le rapport molaire [agent polaire/ métal alcalin de l'amorceur anionique] peut aller jusqu'à 3, avantageusement jusqu'à 2.

Lorsque l'agent polaire est une tétraalkyldiamine, telle que la TMEDA, et l'amorceur anionique un alkyllithien, le rapport molaire [agent polaire/ lithium de l'amorceur anionique] est avantageusement supérieur à 0,3, plus avantageusement supérieur à 0,4. Le rapport molaire [agent polaire/ lithium de l'amorceur anionique] peut avantageusement aller jusqu'à 3, plus avantageusement jusqu'à 2.

### I.1.3 Température de polymérisation

Dans le procédé selon l'invention, la température de polymérisation est inférieure à 80°C. En effet, on a constaté qu'au-delà de cette température, le taux d'insertion du monomère diénique cyclique est faible, le copolymère obtenu comprenant, par rapport au nombre total en mole d'unités, moins de 5% en moles d'unités issues de ce monomère diénique cyclique.

La température de polymérisation est avantageusement inférieure à 75°C, plus avantageusement inférieure à 70°C.

La température de polymérisation varie avantageusement de 45°C à 70°C, plus avantageusement de 50°C à 60°C.

### 1.1.4 Autres paramètres du procédé

La polymérisation est de préférence effectuée en présence d'un solvant hydrocarboné inerte qui peut être par exemple un hydrocarbure aliphatique ou alicyclique comme le pentane, l'hexane, l'heptane, l'iso-octane, le cyclohexane, le méthylcyclohexane ou un hydrocarbure aromatique comme le benzène, le toluène, le xylène.

### 1.1.5 Etape post-polymérisation

La réaction de polymérisation permet de préparer un polymère diénique vivant.

Selon une variante de l'invention, la réaction de polymérisation est ensuite stoppée par la désactivation des chaînes vivantes de manière conventionnelle. Afin de stopper la réaction de polymérisation, il est connu de l'homme de l'art d'introduire un composé protique. A titre d'exemple, on peut citer les alcools tels que le méthanol, les phénols tels que l'hydroquinone ou le résorcinol, les hydroxylamines telles que la N,N-diethylhydroxylamine.

Selon une autre variante de l'invention, le polymère vivant issu de la réaction de polymérisation peut ensuite être modifié pour préparer un polymère fonctionnalisé, couplé ou étoilé selon la nature de l'agent de modification utilisé. Cette modification post-polymérisation est effectuée de manière connue en soi par addition d'un agent de fonctionnalisation, de couplage et/ou d'étoilage sur les chaînes polymériques vivantes ou inversement.

Les agents de fonctionnalisations peuvent par exemple introduire une ou plusieurs fonctions non-polaires au sein du polymère. De tels agents sont connus en soi tels que par exemple Me2SiCI2, MeSiCl3, SiCl4, le 1,6-bis-trichlorosilylhexane, Bu2SnCI2, SnCl4... Ce type de fonctionnalisation améliore par exemple dans une composition pour pneumatique l'interaction entre la charge et l'élastomère ou encore certaines propriétés de l'élastomère fonctionnalisé.

Les agents de fonctionnalisation peuvent également introduire une ou plusieurs fonctions polaires au sein du polymère. La fonction polaire peut être choisie par exemple parmi les fonctions de type amine, silanol, époxyde, éther, ester, hydroxyl, acide carboxylique, alkoxysilane, alkoxysilane substitué par une autre fonction, notamment amine ou thiol, ... Ces fonctions améliorent notamment dans une composition pour pneumatique l'interaction entre une charge inorganique et l'élastomère.

Il est possible, d'obtenir un mélange de chaînes polymères portant des fonctions différentes en faisant réagir successivement différents agents de fonctionnalisation. Par exemple, il est possible de faire réagir dans un premier temps les chaînes vivantes avec un agent introduisant une fonction non-polaire à base de silicium ou d'étain, puis de faire réagir les chaînes vivantes restantes avec un agent de fonctionnalisation introduisant une fonction polaire.

Le groupement issu de la modification post-polymérisation peut se situer en bout de chaîne. On dira alors que le polymère est fonctionnalisé en extrémité de chaîne. C'est généralement un polymère obtenu par réaction d'un élastomère vivant sur une molécule au moins monofonctionnelle pour réagir avec un bout de chaîne vivant, la fonction étant tout type de groupement chimique connu par l'homme de l'art, notamment tel qu'évoqué plus haut.

Le groupement issu de la modification post-polymérisation peut se situer dans la chaîne élastomère principale. On dira alors que le polymère est couplé. C'est généralement un polymère obtenu par réaction d'un polymère vivant sur un agent de couplage, c'est à dire toute molécule au moins difonctionnelle pour réagir avec un bout de chaîne vivant, la fonction étant tout type de groupement chimique connu par l'homme de l'art notamment tel qu'évoqué plus haut.

Le groupement issu de la modification post-polymérisation peut être central auquel n chaînes ou branches polymères (n>2) sont liées formant une structure en étoile du polymère. On dira alors que le polymère est étoilé à n branches. C'est généralement un polymère obtenu par réaction d'un polymère vivant sur un agent d'étoilage, c'est à dire toute molécule multifonctionnelle pour réagir avec un bout de chaîne vivant, la fonction étant tout type de groupement chimique connu par l'homme de l'art notamment tel qu'évoqué plus haut.

### 1.1.6 Etape pré-polymérisation

Préalablement à l'étape de polymérisation, le procédé selon l'invention peut comprendre une étape de synthèse du méthylène de cyclopentène à partir du myrcène par métathèse de fermeture de cycle.

Le myrcène est un monoterpène accessible à partir des plantes. Il peut être extrait d'huiles essentielles de divers plantes telles que le pin, le genévrier ou encore la menthe. Il s'agit donc d'une matière première renouvelable non pétrochimique favorable au développement durable.

Le procédé selon l'invention peut être mis en œuvre de manière continue ou discontinue.

Le copolymère obtenu par le procédé selon l'invention est avantageusement un élastomère.

### I.2 Copolymère selon l'invention

La présente invention a également pour objet un copolymère statistique comprenant, réparties de manière statistique au sein de la chaine linéaire principale du copolymère, des unités insaturées issues d'au moins un monomère diénique acyclique et des unités cycliques issues d'au moins un monomère diénique cyclique dont une double liaison C=C est endocyclique et conjuguée à une double liaison C=C exocyclique.

En particulier, ce copolymère est susceptible d'être obtenu par le procédé selon l'invention.

Le copolymère statistique pourra également comprendre des unités issues d'un autre monomère. Le copolymère selon l'invention comprend des unités issues des monomères décrits précédemment. Il est bien connu que le diène conjugué acyclique peut s'insérer selon plusieurs motifs dits 1,2 ou 1,4 ou sous la forme d'un cycle. Le diène conjugué cyclique peut lui aussi s'insérer selon des motifs 1,2 ou 1,4. En particulier, le diène conjugué cyclique s'insère sous la forme d'un cycle comprenant une double liaison endocyclique ou sous la forme d'un cycle comprenant une double liaison exocyclique.

Le copolymère est avantageusement un élastomère.

Dans le copolymère selon l'invention, le pourcentage molaire d'unités issues du monomère diénique cyclique, par rapport au nombre total d'unités, est avantageusement d'au moins 5%, plus avantageusement d'au moins 10%. Dans le copolymère selon l'invention, le pourcentage molaire d'unités issues du monomère diénique cyclique, par rapport au nombre total d'unités, est avantageusement inférieur à 50%, plus avantageusement inférieur à 30%.

Dans le copolymère selon l'invention, le pourcentage molaire d'unités issues du monomère diénique acyclique, par rapport au nombre total d'unités, est avantageusement d'au moins 50%, plus avantageusement d'au moins 70%. Dans le copolymère selon l'invention, le pourcentage molaire d'unités issues du monomère diénique acyclique, par rapport au nombre total d'unités, est avantageusement inférieur à 95%, plus avantageusement inférieur à 90%.

Le copolymère selon l'invention présente une température de transition vitreuse Tg comprise entre 0°C et -70°C.

### I.3 Compositions selon l'invention

La présente invention a également pour objet une composition comprenant le copolymère selon l'invention. La composition est avantageusement une composition de caoutchouc, en particulier une composition utilisable dans la fabrication d'un pneumatique.

La composition peut également comprendre un ou plusieurs des composants suivants :
- une charge nanométrique ou renforçante,
- un système de réticulation,
- un deuxième élastomère ou caoutchouc « diénique »,
- divers additifs usuellement présents dans les compositions pour pneumatiques.

La présente invention a également pour objet un pneumatique dont un de ses éléments constitutifs comprend une composition selon l'invention.

### II- EXEMPLES DE REALISATION DE L'INVENTION

Les polymères sont caractérisés, avant cuisson, comme indiqué ci-après.

### Chromatographie d'exclusion stérique :

On utilise la chromatographie d'exclusion stérique ou SEC (Size Exclusion Chromatography). La SEC permet de séparer les macromolécules en solution suivant leur taille à travers des colonnes remplies d'un gel poreux. Les macromolécules sont séparées suivant leur volume hydrodynamique, les plus volumineuses étant éluées en premier.

Sans être une méthode absolue, la SEC permet d'appréhender la distribution des masses molaires d'un polymère. A partir de produits étalons commerciaux, les différentes masses molaires moyennes en nombre (Mn) et en poids (Mw) peuvent être déterminées et l'indice de polymolécularité (Ip = Mw/Mn) calculé via un étalonnage dit de MOORE.

Préparation du polymère: Il n'y a pas de traitement particulier de l'échantillon de polymère avant analyse. Celui-ci est simplement solubilisé, dans du (tétrahydrofuranne + 1 %vol. de diisopropylamine + 1 %vol. de triéthylamine + 1 %vol. d'eau distillée) ou dans du chloroforme, à une concentration d'environ 1 g/l. Puis la solution est filtrée sur filtre de porosité 0,45µm avant injection.

Analyse SEC: L'appareillage utilisé est un chromatographe « WATERS alliance ». Le solvant d'élution est du tétrahydrofuranne + 1 %vol. de diisopropylamine + 1 %vol. de triéthylamine ou du chloroforme selon le solvant utilisé pour la mise en solution du polymère. Le débit est de 0,7 ml/min, la température du système de 35°C et la durée d'analyse de 90 min. On utilise un jeu de quatre colonnes WATERS en série, de dénominations commerciales « STYRAGEL HMW7 », « STYRAGEL HMW6E » et deux « STYRAGEL HT6E ».

Le volume injecté de la solution de l'échantillon de polymère est 100 µl. Le détecteur est un réfractomètre différentiel « WATERS 2410 » et le logiciel d'exploitation des données chromatographiques est le système « WATERS EM POWER».

Les masses molaires moyennes calculées sont relatives à une courbe d'étalonnage réalisée à partir de polystyrènes étalons commerciaux « PSS READY CAL-KIT ».

### Température de transition vitreuse :

Les températures de transition vitreuse Tg des polymères sont mesurées au moyen d'un calorimètre différentiel (" differential scanning calorimeter "). L'analyse est réalisée selon les requis de la norme ASTM D3418-08 (2008).

### Détermination de la microstructure par spectroscopie RMN ¹H et ¹³C :

Les déterminations des taux des différentes unités monomères et de leurs microstructures au sein du copolymère sont réalisées par une analyse RMN. Les spectres sont acquis sur un spectromètre 500 MHz BRUKER AVANCE équipé d'une sonde " Broad Band " BBIz-grad 5 mm. D'abord, la caractérisation des espèces est obtenue à partir d'expériences 1D 1H et 2D de corrélation 1H-13C HSQC et HMBC. Ensuite, les microstructures sont déterminées à partir de spectres 13C découplés ¹H Les échantillons sont solubilisés dans le chloroforme CDCl₃.

L'attribution spectrale des signaux ¹H et ¹³C est répertoriée tableaux ci-dessous :

| δ ¹H (ppm) | Nombre de 1H | Attribution |
|---|---|---|
| 4,5 à 4,8 | 2 | = **CH2** du méthylènecyclopentène |
| 4,8 à 5,1 | 2+2 | PB1,2 + Cycles PB |
| 5,1 à 5,6 | 2+1 | PB1,4 + PB-1,2 |
| 5,6 à 6,0 | 1 | Cycles PB |
| δ ¹³C (ppm) | Nombre de 13C | Attribution |
| 104,0 à 108,7 | 1 | = **CH2** du méthylènecyclopentène |
| 111,7 à 113,1 | 1 | **CH2**=CH cycles PB |
| 113,1 à 117,8 | 1 | **CH2**=CH PB1,2 |
| 124,6 à 133,2 | 2 | **CH** PB1,4 |
| 139,3 à 146,1 | 1 + 1 | CH2=**CH** cycles PB + CH2=**CH** PB1,2 |
| 153,1 à 158,8 | 1 | **C quaternaire** du méthylènecyclopentène |

### Taux de conversion des monomères :

Le taux de conversion des monomères est déterminé à 24 heures par pesée d'un extrait séché à 140°C sous pression réduite de 200 mmHg. Il est exprimé en pourcentage.

### Exemple 1 : Copolymère statistique de 1,3-butadiène et 3-Méthylènecyclopentène (proportion massique 70/30)

Dans un réacteur de 250 millilitres, maintenu sous une pression d'azote de 2 bars, contenant 61 millilitres de méthylcyclohexane, sont ajoutés 0,48 millilitres (soit 0,5 équivalents par rapport au lithium actif) d'une solution de tetraméthyléthylènediamine (TMEDA) à 0,1mol/L dans le méthylcyclohexane, ainsi que 2g de 3-méthylènecyclopentène (MCP) et 4,7g de butadiène (BTD). Après neutralisation des impuretés dans la solution à polymériser par addition de s-butyllithium, sont ajoutés 1,12 millilitres de s-butyllithium à 0,085mol/L. Le milieu réactionnel prend alors une coloration jaune. La polymérisation est conduite à 50 °C.
Après 24 heures, le taux de conversion des monomères atteint 86%.

La polymérisation est stoppée par ajout d'un excès de méthanol par rapport au lithium : une décoloration totale du milieu réactionnel est observée. La solution de polymère est soumise à un traitement antioxydant par addition de 0,4 parties pour cent parties d'élastomères (pce) d'une solution à 100g/L dans le toluène de 4,4'-méthylène-bis-2,6-tert-butylphénol et de N-(1,3-diméthylbutyl)-N'-phényl-p-phénylènediamine dans les proportions massiques 80/20, puis le polymère est séché par étuvage à 50°C sous vide/azote pendant deux jours.

La masse moléculaire relative Mn de ce copolymère est de 31700g/mol et l'Ip est de 1,07. La microstructure est la suivante (PB = polybutadiène) : 43,7%ₘₒₗₐᵢᵣₑ de PB-1,4 sur l'ensemble du copolymère, 43,4%ₘₒₗₐᵢᵣₑ de PB-1,2 sur l'ensemble du copolymère et 12,3%ₘₒₗₐᵢᵣₑ de MCP sur l'ensemble du copolymère.

La température de transition vitreuse Tg de ce copolymère est de -47°C.

En suivant ce protocole mais en faisant varier la température de polymérisation et/ou le nombre d'équivalents molaire en TMEDA, par rapport au lithium de l'amorceur, on prépare d'autres copolymères.

Les caractéristiques des copolymères synthétisés sont reportées dans le tableau suivant :

**Tableau 1**

| Conditions opératoires | | Résultats RMN ¹³C | | | | Conversion / DSC | |
|---|---|---|---|---|---|---|---|
| T° (°C) | Equivalents de TMEDA | %molaire PB 1,4/(PB+MCP) | %molaire PB 1,2/(PB+MCP) | %molaire PB cycle/(PB+MCP) | %molaire MCP/(PB+MCP) | %Conv | Tg (°C) |
| 50 | 0,5 | 43,7 | 43,4 | 0,6 | 12,3 | 86 | -47 |
| | 1 | 29,2 | 54,9 | 2 | 13,9 | 91 | -29 |
| | 2 | 21,1 | 62,2 | 1,9 | 14,8 | 90 | -20 |
| 60 | 2 | 26,7 | 57,3 | 1,6 | 14,4 | 87 | -24 |
| 70 | 1 | 39,5 | 46,6 | 1,5 | 12,4 | 85 | -42 |
| 90* | 0,4 | 66,3 | 28,2 | 1,5 | 4,0 | 74 | -72 |

| | | | | | | | |
|---|---|---|---|---|---|---|---|
| * hors invention | | | | | | | |

On constate que lorsque la température de polymérisation augmente, le taux d'insertion en 3-méthylènecyclopentène diminue.

### Exemple 2 : Copolymère statistique de 1,3-butadiène et 3-Méthylènecyclopentène (proportion massique 50/50 et 2eq/Li d'agent polaire)

Dans un réacteur de 250 millilitres, maintenu sous une pression d'azote de 2 bars, contenant 61 millilitres de méthylcyclohexane, sont ajoutés 1,14 millilitres (soit 2 équivalents par rapport au lithium actif) d'une solution de tetraméthyléthylènediamine (TMEDA) à 0,1mol/L dans le méthylcyclohexane, ainsi que 2g de 3-méthylènecyclopentène (MCP) et 2g de butadiène (BTD). Après neutralisation des impuretés dans la solution à polymériser par addition de s-butyllithium, sont ajoutés 0,67 millilitres de s-butyllithium à 0,085mol/L. Le milieu réactionnel prend alors une coloration jaune. La polymérisation est conduite à 50 °C.
Après 24 heures, le taux de conversion des monomères atteint 96%.
La polymérisation est stoppée par ajout d'un excès de méthanol par rapport au lithium : une décoloration totale du milieu réactionnel est observée. La solution de polymère est soumis à un traitement antioxydant par addition de 0,4 parties pour cent parties d'élastomères (pce) d'une solution à 100g/L dans le toluène de 4,4'-méthylène-bis-2,6-tert-butylphénol et de N-(1,3-diméthylbutyl)-N'-phényl-p-phénylènediamine dans les proportions massiques 80/20, puis le polymère est séché par étuvage à 50°C sous vide/azote pendant deux jours.
La masse moléculaire relative Mn de ce copolymère est de 30550g/mol et l'Ip est de 1,33. La microstructure est la suivante : 15,4%ₘₒₗₐᵢᵣₑ de PB-1,4 sur l'ensemble du copolymère, 50,4%ₘₒₗₐᵢᵣₑ de PB-1,2 sur l'ensemble du copolymère, 1,86%ₘₒₗₐᵢᵣₑ cycle PB sur l'ensemble du copolymère et 32,3%ₘₒₗₐᵢᵣₑ de MCP sur l'ensemble du copolymère.
La température de transition vitreuse Tg de ce copolymère est de -13°C.

### Exemple 3 : Copolymère statistique de 1,3-butadiène et 3-Méthylènecyclopentène (proportion massique 50/50 et 0,4 eq/Li d'agent polaire)

Dans un réacteur de 250 millilitres, maintenu sous une pression d'azote de 2 bars, contenant 61 millilitres de méthylcyclohexane, sont ajoutés 0,23 millilitres (soit 0,4 équivalents par rapport au lithium actif) d'une solution de tetraméthyléthylènediamine (TMEDA) à 0,1mol/L dans le méthylcyclohexane, ainsi que 2g de 3-méthylènecyclopentène (MCP) et 2g de butadiène (BTD). Après neutralisation des impuretés dans la solution à polymériser par addition de s-butyllithium, sont ajoutés 0,67 millilitres de s-butyllithium à 0,085mol/L. Le milieu réactionnel prend alors une coloration jaune. La polymérisation est conduite à 50 °C.
Après 24 heures, le taux de conversion des monomères atteint 84%.
La polymérisation est stoppée par ajout d'un excès de méthanol par rapport au lithium : une décoloration totale du milieu réactionnel est observée. La solution de polymère est soumis à un traitement antioxydant par addition de 0,4 parties pour cent parties d'élastomères (pce) d'une solution à 100g/L dans le toluène de 4,4'-méthylène-bis-2,6-tert-butylphénol et de N-(1,3-diméthylbutyl)-N'-phényl-p-phénylènediamine dans les proportions massiques 80/20, puis le polymère est séché par étuvage à 50°C sous vide/azote pendant deux jours.
La masse moléculaire relative Mn de ce copolymère est de 26700g/mol et l'Ip est de 1,3. La microstructure est : 41,5%ₘₒₗₐᵢᵣₑ de PB-1,4 sur l'ensemble du copolymère, 37,1 %ₘₒₗₐᵢᵣₑ de PB-1,2 sur l'ensemble du copolymère, et 21,4%ₘₒₗₐᵢᵣₑ de MCP sur l'ensemble du copolymère.
La température de transition vitreuse Tg de ce copolymère est de -44°C.

### Exemple comparatif 1: Copolymérisation de 1,3-butadiène et 3-Méthylènecyclopentène (proportion massique 70/30 et sans agent polaire)

Dans un réacteur de 250 millilitres, maintenu sous une pression d'azote de 2 bars, contenant 61 millilitres de méthylcyclohexane, sont ajoutés 2g de 3-Méthylènecyclopentène (MCP) et 4,7g de butadiène (BTD). Après neutralisation des impuretés dans la solution à polymériser par addition de s-butyllithium, sont ajoutés 1,12 millilitres de s-butyllithium à 0,085mol/L. Le milieu réactionnel prend alors une coloration jaune. La polymérisation est conduite à 50 °C.
Après 24 heures, le taux de conversion des monomères atteint 65%.
La polymérisation est stoppée par ajout d'un excès de méthanol par rapport au lithium : une décoloration totale du milieu réactionnel est observée. La solution de polymère est soumis à un traitement antioxydant par addition de 0,4 parties pour cent parties d'élastomères (pce) d'une solution à 100g/L dans le toluène de 4,4'-méthylène-bis-2,6-tert-butylphénol et de N-(1,3-diméthylbutyl)-N'-phényl-p-phénylènediamine dans les proportions massiques 80/20, puis le polymère est séché par étuvage à 50°C sous vide/azote pendant deux jours.
La masse moléculaire relative Mn de ce polymère est de 29500g/mol et l'Ip est de 1,15.

La microstructure, déterminée par spectroscopie RMN ¹³C, est la suivante : 93,2%ₘₒₗₐᵢᵣₑ de PB-1,4 sur l'ensemble du copolymère, 6,8%ₘₒₗₐᵢᵣₑ de PB-1,2 sur l'ensemble du copolymère et 0%ₘₒₗₐᵢᵣₑ de MCP sur l'ensemble du copolymère.
La température de transition vitreuse Tg de ce polymère est de -73°C.

### Exemple comparatif 2: Copolymérisation de 1,3-butadiène et de 1,3-cyclohexadiène (proportion massique 63/37 et 1,7 eq/Li d'agent polaire)

Dans un réacteur de 250 millilitres, maintenu sous une pression d'azote de 2 bars, contenant 54 millilitres de toluène, sont ajoutés 3,18 millilitres (soit 1,7 équivalents par rapport au lithium actif) d'une solution de 1,4- diazabicyclo[2,2,2]octane (DABCO) à 0,214 mol/L dans le méthylcyclohexane, ainsi que 2,2g de 1,3-cyclohexadiène et 3,8g de butadiène. Après neutralisation des impuretés dans la solution à polymériser par addition de s-butyllithium, sont ajoutés 3,92 millilitres de s-butyllithium à 0,102 mol/L. Le milieu réactionnel prend alors une coloration jaune. La polymérisation est conduite à 25 °C.

Après 1,5 heures, le taux de conversion des monomères atteint 62% (échantillon 1). Après 24 heures, le taux de conversion des monomères atteint 99% (échantillon 2). La polymérisation est stoppée par ajout d'un excès de méthanol par rapport au lithium : une décoloration totale du milieu réactionnel est observée. La solution de polymère est soumis à un traitement antioxydant par addition de 0,4 parties pour cent parties d'élastomères (pce) d'une solution à 100g/L dans le toluène de 4,4'-méthylène-bis-2,6-tert-butylphénol et de N-(1,3-diméthylbutyl)-N'-phényl-p-phénylènediamine dans les proportions massiques 80/20, puis le polymère est séché par étuvage à 50°C sous vide/azote pendant deux jours.

**¹ Déterminé par spectroscopie RMN¹H. ² Déterminés par chromatographie SEC.**

| | Taux de conversion en monomère (%) | Taux de butadiène-1,4 (%molaire)¹ | Taux de butadiène-1,2 (%molaire)¹ | Taux de cyclohexadiène (%molaire)¹ | Masse moléculaire Mn (g/mol) (Ip)² |
|---|---|---|---|---|---|
| Echantillon 1 | 62 | 55 | 45 | 0 | 9500 (1,03) |
| Echantillon 2 | 99 | 41 | 34 | 25 | 13000 (1,08) |

L'insertion du cyclohexadiène a lieu majoritairement en fin de polymérisation. Elle n'est donc pas statistique. Un copolymère à blocs est obtenu.

## Revendications

1. Procédé de préparation d'un copolymère statistique à base de monomères diéniques acycliques et de monomères diéniques cycliques, **caractérisé en ce qu'**il comprend une étape de copolymérisation, en présence d'un agent polaire et d'un amorceur anionique dans un solvant de polymérisation, d'au moins un monomère diénique acyclique et d'au moins un monomère diénique cyclique dont une double liaison C=C est endocyclique et conjuguée à une double liaison C=C exocyclique, à une température de polymérisation inférieure à 80°C, le rapport molaire [agent polaire/ fonction de l'amorceur anionique] étant supérieur à 0,1.

2. Procédé selon la revendication 1, **caractérisé en ce que** le rapport molaire [agent polaire/ fonction de l'amorceur anionique] est supérieur ou égal à 0,3.

3. Procédé selon l'une quelconque des revendications précédentes, **caractérisé en ce que** le monomère diénique acyclique est un monomère diénique conjugué, de préférence le butadiène-1,3 ou l'isoprène.

4. Procédé selon l'une quelconque des revendications précédentes, **caractérisé en ce que** le monomère diénique cyclique est un monomère répondant à la formule (I) suivante : 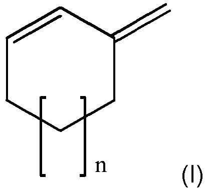 Où n=0 ou 1,
de préférence le 3-méthylènecyclopentène.

5. Procédé selon l'une quelconque des revendications précédentes, **caractérisé en ce qu'**on copolymérise également un composé vinylaromatique ayant de 8 à 20 atomes de carbone, avantageusement le styrène.

6. Procédé selon l'une quelconque des revendications précédentes, **caractérisé en ce que** au moins l'une des conditions suivantes est respectée :
- l'agent polaire est une tétraalkyldiamine, notamment, la N,N,N',N'-tétraméthyléthylènediamine ;
- l'amorceur anionique contient un métal alcalin, de préférence l'amorceur anionique est un organolithien ;
- la température de polymérisation varie de 45°C à 70°C, de préférence de 50°C à 60°C.

7. Copolymère statistique comprenant, réparties de manière statistique au sein de la chaine linéaire principale du copolymère, des unités insaturées issues d'au moins un monomère diénique acyclique et des unités cycliques issues d'au moins un monomère diénique cyclique dont une double liaison C=C est endocyclique et conjuguée à une double liaison C=C exocyclique.

8. Copolymère selon la revendication précédente **caractérisé en ce que** le monomère diénique acyclique est un monomère diénique conjugué, de préférence le butadiène-1,3 ou l'isoprène.

9. Copolymère selon l'une quelconque des revendications 7 à 8 **caractérisé en ce que** le monomère diénique cyclique est un monomère répondant à la formule (I) suivante : 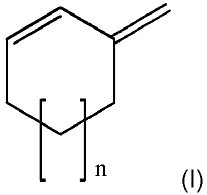 Où n=0 ou 1
de préférence le 3-méthylènecyclopentène.

10. Copolymère selon l'une quelconque des revendications 7 à 9 **caractérisé en ce qu'**il comprend également des unités issues d'un composé vinylaromatique ayant de 8 à 20 atomes de carbone, avantageusement du styrène.

11. Copolymère selon l'une quelconque des revendications 7 à 10 **caractérisé en ce que** le pourcentage molaire d'unités issues du monomère diénique cyclique, par rapport au nombre total d'unités, est d'au moins 5%, avantageusement d'au moins 10%.

12. Copolymère selon l'une quelconque des revendications 7 à 11 **caractérisé en ce que** le pourcentage molaire d'unités issues du monomère diénique cyclique, par rapport au nombre total d'unités, est inférieur à 50%, avantageusement inférieur à 30%.

13. Copolymère selon l'une quelconque des revendications 7 à 12 ou copolymère susceptible d'être obtenu par la procédé défini selon l'une quelconque des revendications 1 à 6, **caractérisé en ce que** ledit copolymère est un élastomère.

14. Composition comprenant le copolymère défini selon l'une quelconque des revendications 7 à 12 ou le copolymère susceptible d'être obtenu par la procédé défini selon l'une quelconque des revendications 1 à 6.

15. Pneumatique dont un de ses éléments constitutifs comprend une composition selon la revendication précédente.

## Patentansprüche

1. Verfahren zur Herstellung eines statistischen Copolymers auf Basis von acyclischen Dien-Monomeren und cyclischen Dien-Monomeren, **dadurch gekennzeichnet, dass** es einen Schritt der Copolymerisation von mindestens einem acyclischen Dien-Monomer und mindestens einem cyclischen Dien-Monomer, dessen C=C-Doppelbindung endocyclisch ist und mit einer exocyclischen C=C-Doppelbindung konjugiert ist, in Gegenwart eines polaren Agens und eines anionischen Initiators in einem Polymerisationslösungsmittel einer Polymerisationstemperatur von weniger als 80 °C, wobei das Molverhältnis [polares Agens/Funktion des anionischen Initiators] größer als 0,1 ist, umfasst.

2. Verfahren nach Anspruch 1, **dadurch gekennzeichnet, dass** das Molverhältnis [polares Agens/Funktion des anionischen Initiators] größer oder gleich 0,3 ist.

3. Verfahren nach einem der vorhergehenden Ansprüche, **dadurch gekennzeichnet, dass** es sich bei dem acyclischen Dien-Monomer um ein konjugiertes Dien-Monomer, vorzugsweise 1,3-Butadien oder Isopren, handelt.

4. Verfahren nach einem der vorhergehenden Ansprüche, **dadurch gekennzeichnet, dass** es sich bei dem cyclischen Dien-Monomer um ein Monomer der folgenden Formel (I): 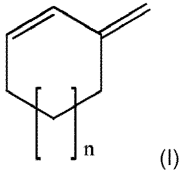 wobei n = 0 oder 1,
vorzugsweise 3-Methylencyclopenten, handelt.

5. Verfahren nach einem der vorhergehenden Ansprüche, **dadurch gekennzeichnet, dass** man außerdem eine vinylaromatische Verbindung mit 8 bis 20 Kohlenstoffatomen, vorteilhafterweise Styrol, copolymerisiert.

6. Verfahren nach einem der vorhergehenden Ansprüche, **dadurch gekennzeichnet, dass** mindestens eine der folgenden Bedingungen erfüllt ist:
- bei dem polaren Agens handelt es sich um ein Tetraalkyldiamin, insbesondere N,N,N',N'-Tetramethylethylendiamin;
- der anionische Initiator enthält ein Alkalimetall, vorzugsweise handelt es sich bei dem anionischen Initiator um eine Organolithiumverbindung;
- die Polymerisationstemperatur variiert von 45 °C bis 70 °C, vorzugsweise von 50 °C bis 60 °C.

7. Statistisches Copolymer, das in statistischer Verteilung in der linearen Hauptkette des Copolymers ungesättigte Einheiten, die sich von mindestens einem acyclischen Dien-Monomer ableiten, und cyclische Einheiten, die sich von mindestens einem cyclischen Dien-Monomer, dessen C=C-Doppelbindung endocyclisch ist und mit einer exocyclischen C=C-Doppelbindung konjugiert ist, ableiten, umfasst.

8. Copolymer nach dem vorhergehenden Anspruch, **dadurch gekennzeichnet, dass** es sich bei dem acyclischen Dien-Monomer um ein konjugiertes Dien-Monomer, vorzugsweise 1,3-Butadien oder Isopren, handelt.

9. Copolymer nach einem der Ansprüche 7 bis 8, **dadurch gekennzeichnet, dass** es sich bei dem cyclischen Dien-Monomer um ein Monomer der folgenden Formel (I): 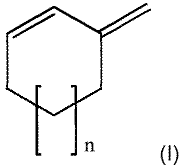 wobei n = 0 oder 1,
vorzugsweise 3-Methylencyclopenten, handelt.

10. Copolymer nach einem der Ansprüche 7 bis 9, **dadurch gekennzeichnet, dass** es außerdem Einheiten, die sich von einer vinylaromatischen Verbindung mit 8 bis 20 Kohlenstoffatomen, vorteilhafterweise Styrol, ableiten, umfasst.

11. Copolymer nach einem der Ansprüche 7 bis 10, **dadurch gekennzeichnet, dass** der Molprozentanteil von Einheiten, die sich von dem cyclischen Dien-Monomer ableiten, bezogen auf die Gesamtzahl von Einheiten, mindestens 5 %, vorteilhafterweise mindestens 10 %, beträgt.

12. Copolymer nach einem der Ansprüche 7 bis 11, **dadurch gekennzeichnet, dass** der Molprozentanteil von Einheiten, die sich von dem cyclischen Dien-Monomer ableiten, bezogen auf die Gesamtzahl von Einheiten, kleiner als 50 %, vorteilhafterweise kleiner als 30 %, ist.

13. Copolymer nach einem der Ansprüche 7 bis 12 oder Copolymer, das durch das gemäß einem der Ansprüche 1 bis 6 definierte Verfahren erhältlich ist, **dadurch gekennzeichnet, dass** es sich bei dem Copolymer um ein Elastomer handelt.

14. Zusammensetzung, umfassend das gemäß einem der Ansprüche 7 bis 12 definierte Copolymer oder das Copolymer, das durch das gemäß einem der Ansprüche 1 bis 6 definierte Verfahren erhältlich ist.

15. Reifen, wobei eines seiner Aufbauelemente eine Zusammensetzung nach dem vorhergehenden Anspruch umfasst.

## Claims

1. Process for preparing a random copolymer based on acyclic diene monomers and on cyclic diene monomers, **characterized in that** it comprises a step of copolymerization, in the presence of a polar agent and an anionic initiator in a polymerization solvent, of at least one acyclic diene monomer and of at least one cyclic diene monomer of which one C=C double bond is endocyclic and conjugated to an exocyclic C=C double bond, at a polymerization temperature below 80°C, the [polar agent/function of the anionic initiator] molar ratio being greater than 0.1.

2. Process according to Claim 1, **characterized in that** the [polar agent/function of the anionic initiator] molar ratio is greater than or equal to 0.3.

3. Process according to either one of the preceding claims, **characterized in that** the acyclic diene monomer is a conjugated diene monomer, preferably 1,3-butadiene or isoprene.

4. Process according to any one of the preceding claims, **characterized in that** the cyclic diene monomer is a monomer corresponding to the
following formula (I): 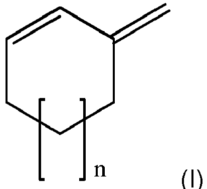 where n = 0 or 1,
preferably 3-methylenecyclopentene.

5. Process according to any one of the preceding claims, **characterized in that** a vinylaromatic compound having from 8 to 20 carbon atoms, advantageously styrene, is also copolymerized.

6. Process according to any one of the preceding claims, **characterized in that** at least one of the following conditions is fulfilled:
- the polar agent is a tetraalkyldiamine, especially N,N,N',N'-tetramethylethylenediamine,
- the anionic initiator contains an alkali metal, preferably the anionic initiator is an organolithium compound,
- the polymerization temperature varies from 45°C to 70°C, preferably from 50°C to 60°C.

7. Random copolymer comprising, randomly distributed within the main linear chain of the copolymer, unsaturated units derived from at least one acyclic diene monomer and cyclic units derived from at least one cyclic diene monomer of which one C=C double bond is endocyclic and conjugated to an exocyclic C=C double bond.

8. Copolymer according to the preceding claim, **characterized in that** the acyclic diene monomer is a conjugated diene monomer, preferably 1,3-butadiene or isoprene.

9. Copolymer according to either one of Claims 7 and 8, **characterized in that** the cyclic diene monomer is a monomer corresponding to the following formula (I): 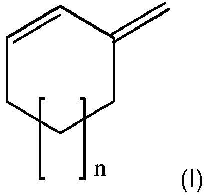 where n = 0 or 1,
preferably 3-methylenecyclopentene.

10. Copolymer according to any one of Claims 7 to 9, **characterized in that** it also comprises units derived from a vinylaromatic compound having from 8 to 20 carbon atoms, advantageously styrene.

11. Copolymer according to any one of Claims 7 to 10, **characterized in that** the molar percentage of units derived from the cyclic diene monomer, relative to the total number of units, is at least 5%, advantageously at least 10%.

12. Copolymer according to any one of Claims 7 to 11, **characterized in that** the molar percentage of units derived from the cyclic diene monomer, relative to the total number of units, is less than 50%, advantageously less than 30%.

13. Copolymer according to any one of Claims 7 to 12 or copolymer capable of being obtained by the process defined according to any one of Claims 1 to 6, **characterized in that** said copolymer is an elastomer.

14. Composition comprising the copolymer defined according to any one of Claims 7 to 12 or copolymer capable of being obtained by the process defined according to any one of Claims 1 to 6.

15. Tyre, of which one of its constituent elements comprises a composition according to the preceding claim.
